# EUROPÄISCHE PATENTSCHRIFT

(11) **EP 1 148 942 B1**
(45) Veröffentlichungstag und Bekanntmachung des Hinweises auf die Patenterteilung: **23.10.2002**
(21) Anmeldenummer: 00909002.8
(22) Anmeldetag: 04.02.2000
(51) Int. Cl.: B01J 19/00, B01L 3/00

(54) **DURCHFLUSSEINRICHTUNG SOWIE IHRE VERWENDUNG ZUM BINDEN VON POLYMEREN AN MEMBRANOBERFLÄCHEN**
FLOW-THROUGH DEVICE AND ITS USE FOR BINDING POLYMERS TO MEMBRANE SURFACES
DISPOSITIF D'ECOULEMENT ET SON UTILISATION POUR FIXER DES POLYMERES SUR DES SURFACES MEMBRANAIRES

(30) Priorität: 05.02.1999 DE 19904784
(43) Veröffentlichungstag der Anmeldung: 31.10.2001
(73) Patentinhaber: Deutsches Krebsforschungszentrum Stiftung des öffentlichen Rechts, 69120 Heidelberg (DE)
(72) Erfinder: MATYSIAK, Stefan, D-69118 Heidelberg (DE)
(74) Vertreter: Schüssler, Andrea, Dr.
(86) Internationale Anmeldenummer: DE0000365
(87) Internationale Veröffentlichungsnummer: WO00045950

(56) Entgegenhaltungen:
- GB-A- 2 246 081
- US-A- 4 493 815
- US-A- 4 990 442
- US-A- 5 108 603
- US-A- 5 624 815

## Beschreibung

Die vorliegende Erfindung betrifft eine Durchflußeinrichtung sowie ihre Verwendung zum Binden von Polymeren an Membranoberflächen, deren Position dort über x,y-Parameter festgelegt wird. Insbesondere wird diese Durchflußeinrichtung bei einem Verfahren zur Synthese von membrangebundenen Molekülbibliotheken eingesetzt.

Die Festphasensynthese von Oligomeren oder kleineren organischen Verbindungen an quellbaren oder nicht quellbaren Trägermaterialien findet meistens an Harzen aus relativ inerten Polymeren (z.B. hochvernetztem Polystyrol) statt, die zu kleinen monodispersen Kügelchen oder zu Pulvern mit einer definierten Zahl von funktionellen Gruppen an ihrer Oberfläche gefertigt sind. Nach Abschluß der Synthese bzw. entsprechender Entschützungsprozeduren, die in getrennten Reaktionskammern stattfinden, können diese Produkte in geeigneten Gefäßen aufgefangen werden. Das ursprüngliche Raster, also die Anordnung bei der Synthese geht dabei verloren oder muß durch aufwendige Maßnahmen wiederhergestellt werden, z.B. durch Umpipettieren. Mit dieser Methode ist es damit fast unmöglich ganze Molekülbibliotheken aufzubauen.

Desweiteren gibt es die Synthese von "spatially addressable combinatorial libraries", also Molekülbibliotheken, bei denen die Information über die Sequenz bzw. die durchgeführten chemischen Schritte über die x,y-Anordnung festgelegt sind. Hier ist insbesondere die parallele Synthese von "Molekül-Arrays" nach der SPOT-Methode (Frank, R., Tetrahedron 48, S. 9217-9232, 1992) auf porösen Membranen hervorzuheben, deren hauptsächlicher Nachteil in einem hohen Zeitaufwand sowie in einem nur mangelhaft entwickelten Automatisierungsgrad besteht.

Die Aufgabe der vorliegenden Erfindung besteht deshalb darin, eine Vorrichtung bereitzustellen, mit der eine automatisierbare Methode zum Binden von Polymeren, insbesondere zum Aufbau von Molekülbibliotheken, durchgeführt werden kann.

Die Automatisierung der Synthese von Molekülbibliotheken auf Membranoberflächen erfolgt durch den Einsatz einer erfindungsgemäßen Durchflußeinrichtung.
Durchflußeinrichtungen werden bisher nur zu Filtrationszwecken eingesetzt und sind z.B. von der Firma Schleicher & Schüll, 37582 Dassel erhältlich.

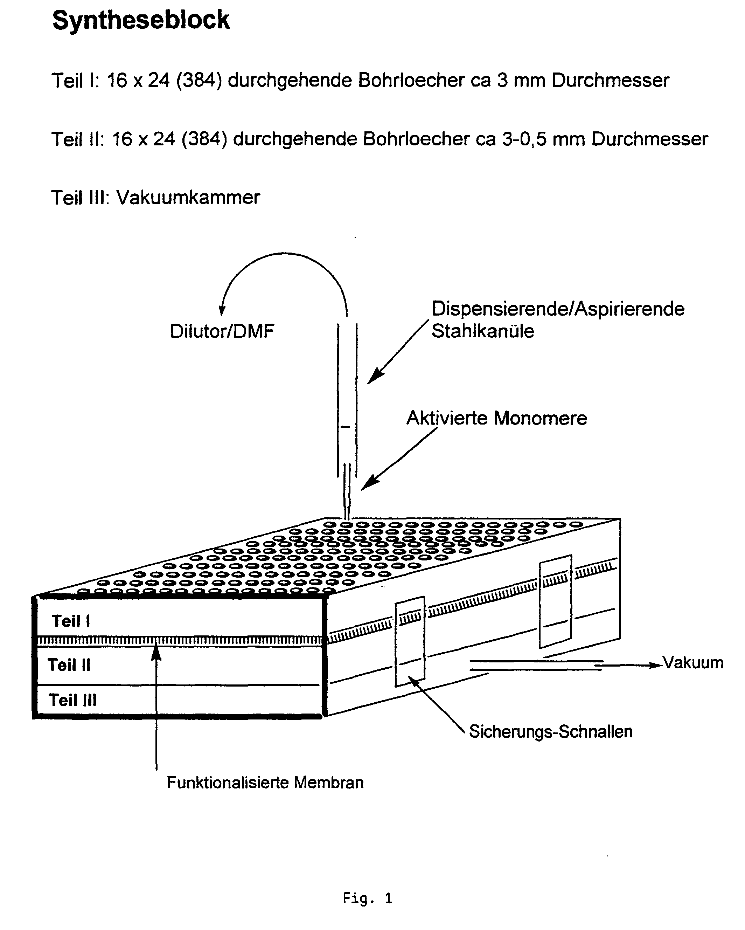

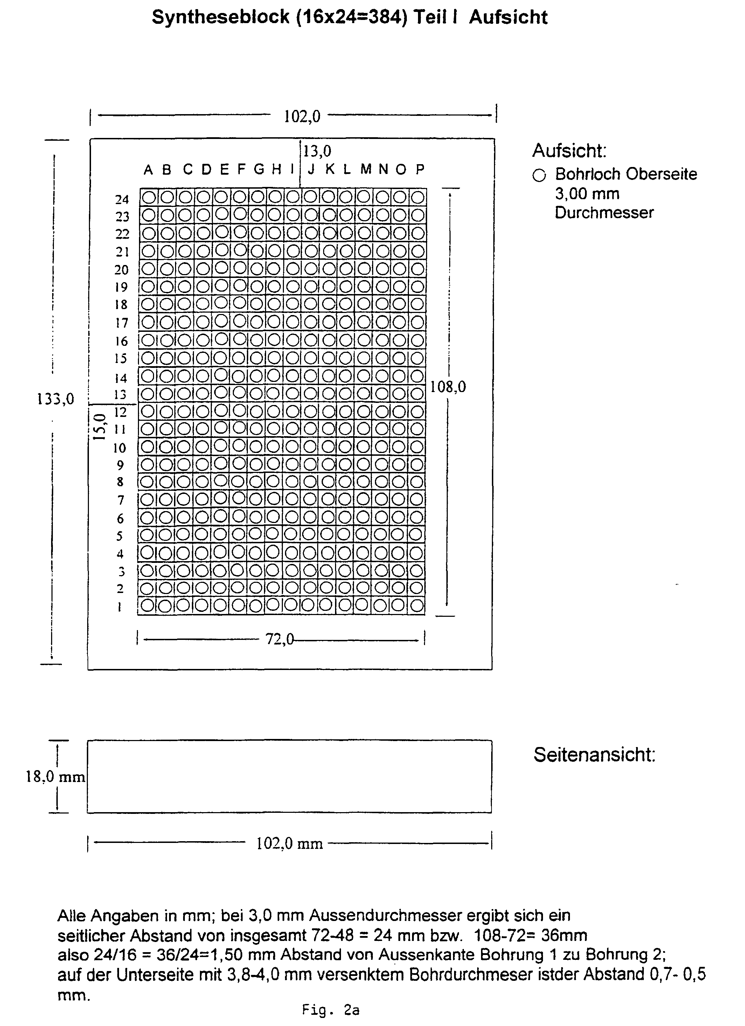

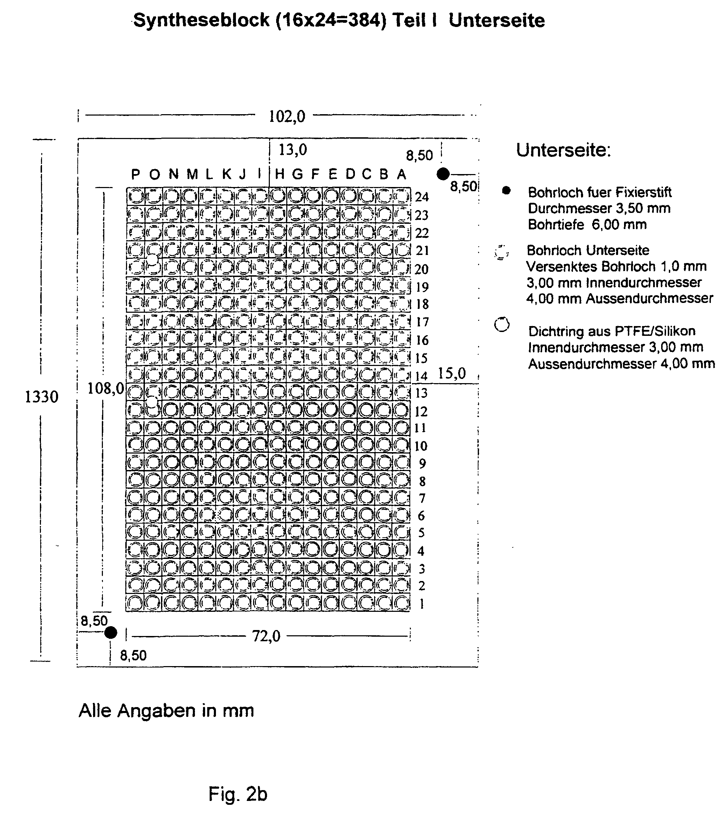

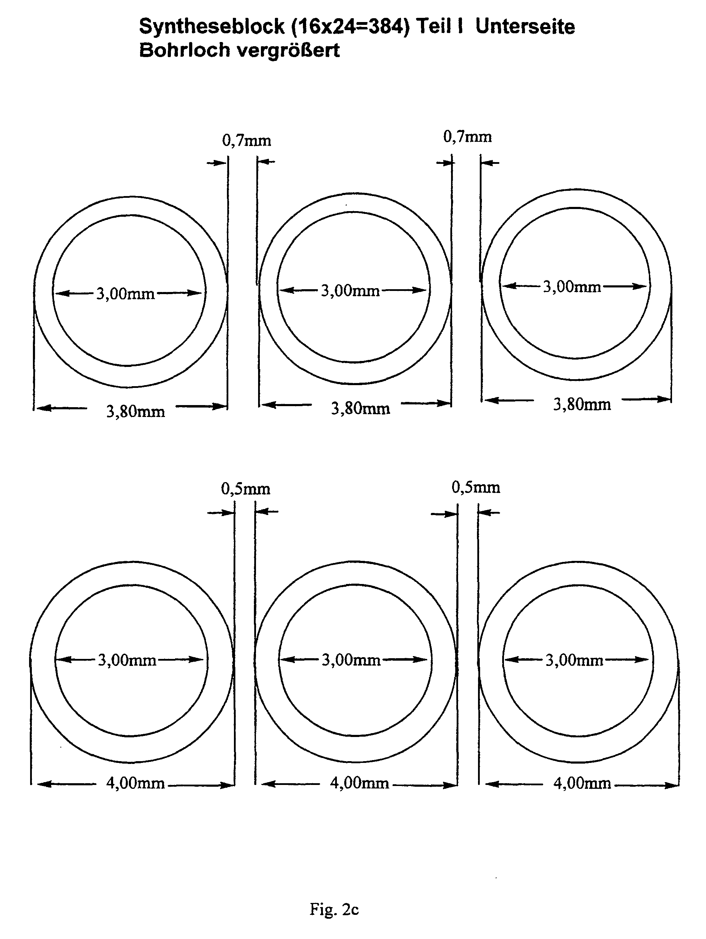

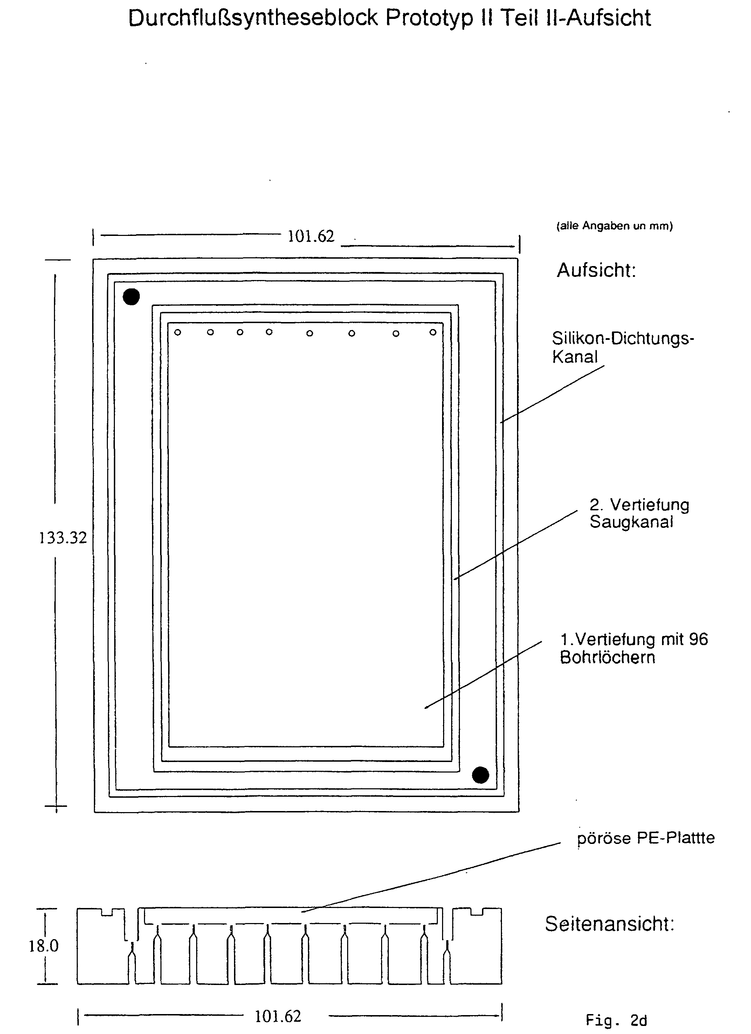

Vorteilhafterweise wird eine Durchflußeinrichtung in Form eines Syntheseblocks gemäß Fig. 1 eingesetzt. Die einzelnen Teile des Syntheseblocks sind in den Fig. 2a - 2e gezeigt. Dieser Syntheseblock zeichnet sich insbesondere dadurch aus, daß ein inertes Material verwendet wird, vorzugsweise Delrin, PTFE oder keramische Materialien.

Der in Fig. 1 gezeigte Syntheseblock zeichnet sich durch einen dreiteiligen Aufbau aus, wobei zwischen Teil I und Teil II eine Synthesemembran angeordnet ist.

Teil I enthält nebeneinander angeordnete Bohrlöcher, die einen Innendurchmesser von ca. 3 mm haben, wobei die BohrlochDurchmesser natürlich jede für die jeweilige Anwendung geeignete Größe haben können. An der Unterseite ist jedes Bohrloch durch einen Dichtring, z.B. aus PFTE/Silikon, abgedichtet. Die Anzahl der Bohrlöcher beträgt mindestens 96, verzugsweise 384 oder mehr.

Teil II weist vorzugsweise folgenden Aufbau auf: Unterhalb der x,y-Löcher von Teil I befindet sich eine grobporige Membran bzw. Platte, z.B. aus PE, PP, PFTE oder Delrin, von mehreren mm Dicke, vorzugsweise 2-15 mm, ganz bevorzugt 4-10 mm, als Unterlage für die vorzugsweise funktionalisierte Synthesemembran, die zwischen Teil I und II zu liegen kommt. Die Porengröße der grobporigen Membran beträgt vorzugsweise von 100 bis 250 µm, bevorzugt 120 bis 200 µm. In einer 1. Vertiefung von Teil II befinden sich eine entsprechende Anzahl von Bohrlöchern, die ebenso wie in Teil I mindestens 96, vorzugsweise 384 oder mehr, beträgt und auch mit jeweils einem Dichtring, z.B. aus PFTE/Silikon, abgedichtet sind. In einer 2. Vertiefung von Teil II wurde zur besseren Durchspülungsund Absaugmöglichkeit ein Saugkanal zur Anlegung eines Vakuums vorgesehen. Durch diesen Aufbau von Teil II, insbesondere durch die grobporige Membran als Unterlage für die Synthesemembran, wird ein angelegtes Vakuum auch auf solche Regionen ausgeweitet, die außerhalb der Dichtungsringe liegen. Das Ansammeln und Verschleppen von unerwünschten Reagenzien wird so verringert. Außerdem ermöglicht diese Modifikation, daß mit einem einzigen Unterbau (Teil II und III) mehrere Reaktortypen (96-Loch, 384-Loch) verwendet werden können.

Teil III enthält eine Vorrichtung in Form mindestens eines Ansaugstücks, um an die Apparatur ein Vakuum anlegen zu können. Ebenso wie die Teile I und II weist Teil III nebeneinander angeordnete Bohrlöcher auf, die vorzugsweise in Anzahl und Größe denen der Teile I und II entsprechen. Eine bevorzugte Modifikation ist das Vorhandensein eines zweiten Vakuumkanals in Teil III, der verhindern soll, daß sich Reagenzien am äußeren Rand der Membran ansammeln. Die beiden Vakuumbereiche (Vakuumkammer unter der Membran, Vakuumkanal für die Randbereiche) sind durch Dichtungen, vorzugsweise aus Silikon, getrennt und können mit verschiedenen Unterdrücken betrieben werden.

Der Zusammenbau der Apparatur erfolgt durch Übereinanderlegen der Teile, wobei zur Arretierung noch Sicherungseinrichtungen, z.B. in Form von Schnallen, vorhanden sind.

Die sich zwischen Teil I und Teil II befindliche Synthesemembran ist eine solche, die sich zum Binden von Polymeren eignet und weist eine Porengröße von 0,1 bis 1,3 µm, bevorzugt 0,2 bis 1,0 µm auf. Eine solche Membran kann aus allen auf diesem Gebiet üblichen Materialien bestehen und sollte vorzugsweise an ihrer Oberfläche funktionelle Gruppen, wie Hydroxyl-, Amino-, Amid-, Phosphat-, Alkyl-, Halogen-, Carboxyl-, Carbonyl-, Thio-, Arylgruppen, Ethengruppen (z.B. Vinyl-, Vinyloxy-, Vinyloxycarbonyl sowie die entsprechenden reinen Thio- bzw. gemischten Thio-Analoga) oder Ethingruppen tragen. Als Grundmaterialien der Membranen eignen sich Nylon, Polyamid, Cellulose, Polypropylen, PFTE, Polyolefin, Polyethylen oder Polystyrol, Polyvinylidenfluorid, Glasfiber, PVC, Polymethylpenten, Polynorbornen-Copolymere (z.B. Topas, Fa. Hoechst). Ganz bevorzugt handelt es sich bei der verwendeten Membran um eine Membran aus oberflächenoxidiertem (hydrophilisiertem) Polypropylen, das entsprechend derivatisiert wurde.

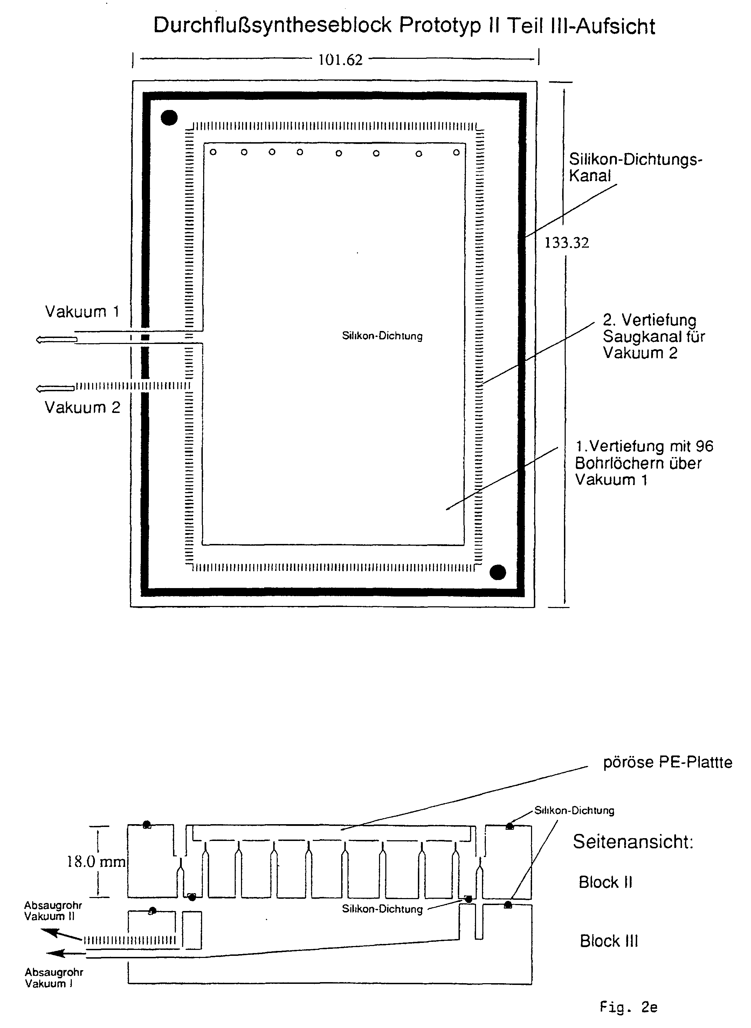

In bevorzugter Weise weist die Membran Kompartimente auf, die einmal automatisch dadurch entstehen, daß die Membran in den Syntheseblock eingespannt wird und durch die Dichtringe an der Unterseite von Teil I des Syntheseblocks Abschnürungen entstehen, die eine seitliche Abgrenzung der runden Reaktionsflächen bewirken. Die seriell aufzutragenden Reagentien, insbesondere die aktivierten Monomere bei der Herstellung einer Molekülbibliothek, werden nun exakt dosiert zugegeben, um laterale Kontaminationen zu vermeiden. Das abgegebene Volumen sollte so bemessen sein, daß die entstehenden Flecken nicht ineinanderlaufen. Alle parallel verlaufenden chemischen Schritte, z.B. Aufbringen der Waschlösungen, erfolgt im Überschuß, d.h. die Chemikalien verteilen sich in allen Reaktionskammern, werden aber durch ein angelegtes Vakuum durch die Unterlage nach unten abgesaugt. Seitlich wird vorzugsweise ein weiteres Vakuum angelegt, um überschüssiges Reagenz abzusaugen (vgl. Fig. 2e).

Andererseits können schon vorher auf der Membran abgeschweißte Bereiche aufgebracht werden, die exakt den Löchern der Teile I und II des Syntheseblocks entsprechen und in diese eingepaßt werden müssen. Dadurch entsteht formal eine den üblichen Syntheseformaten identische Anordnung mit relativ kleinen, gegeneinander abgegrenzten Membranflächen. Dies funktioniert in der Weise, daß die Membran in den Syntheseblock eingelegt wird. Durch Abdrücken der Dichtungsringe und Anlegen eines erhöhten Drucks und/oder Temperatur oder durch Abdichten mit einem inerten thermoplastischen Kunststoff (z.B. niedrig schmelzendes Polypropylengranulat) unterhalb der Dichtungen entstehen einzelne abgetrennte Reaktionskammern. Reagentien, die seriell oder parallel aufgetragen werden sollen, können nun auch im Überschuß aufgebracht werden, ohne daß es zu einer lateralen Kontamination kommt, weil die Membrankompartimente gegeneinander abgedichtet sind.. Es entsteht eine "Spiegelei"-Struktur, deren abgegrenzte Einheiten einen Vorteil hinsichtlich der Vermeidung von Kontaminationen und Verschleppen von Reaktanten über die gesamte Membran.

Reagentien werden über handelsübliche Synthesizer (z.B. Spotsynthesizer der Fa. Abimed Analysentechnik, Langenfeld, Deutschland) in die Löcher von Teil I des Syntheseblocks auf die Membranoberfläche pipettiert und dort kovalent durch eine chemische Reaktion gebunden. Überschüssige Reagentien bzw. Waschlösungen werden mittels Vakuum abgesaugt. Dadurch verkürzt sich die Zykluszeit erheblich, die Bindung der Polymere an der Membranoberfläche und somit der Aufbau einer Molekülbibliothek erfolgt um ein Vielfaches schneller. Neben der minimierten Synthesezeit ermöglicht die nach wie vor vorhandene Membranstruktur, daß die komplette Membran nach beendeter Synthese einem Screening-Verfahren (z.B. Hybridisierung einer radioaktiv markierten DNA-Sonde aufgrund spezifischer Basenpaarung) unterzogen werden kann. Dies findet statt, ohne daß die Oligomeren in andere Gefäße wie im Stand der Technik überführt werden oder auf anderen Trägern verankert werden müssen. Dadurch ist gewährleistet, daß beispielsweise die gesamte Molekülbibliothek unter exakt gleichen Bedingungen auf spezifische Wechselwirkungen hin überprüft werden kann.

Unter Polymeren sollen verzugsweise DNA, RNA, Aminosäuren, Peptide, Proteine, Nukleinsäureanaloga (z.B. PNA, LNA) verstanden werden.

Die Erfindung wird weiter anhand der Figuren beschrieben, welche zeigen:
- Fig. 1:: Syntheseblock
- Fig. 2:: Aufbau des Syntheseblocks
a: Syntheseblock Teil I Aufischt
b: Syntheseblock Teil I Unterseite
c: Syntheseblock Teil I Unterseite, Bohrloch vergrößert
d: Syntheseblock Teil II Aufsicht
e: Syntheseblock Teil III Aufsicht
- Fig. 3:: a: Membran hybridisiert mit d(T)₁₆ ³³PγATP
b: Membran zusätzlich hybridisiert mit d (C)₁₆ ³³PγATP
c: Kontrolle

Die Erfindung wird weiter anhand des Beispiels beschrieben:

### Beispiel

- Membran:: hydrophilisiertes Polypropylen, Porengröße 0,2 µm, umgesetzt mit Trisamin-Jeffamin 500 (bisfunktionelles Amino-Polyethylenglykol) nach Carbonyldiimidazol-Aktivierung Beladung: 0,12 µmol/cm²
- Vorbehandlung:: Membran mit einer Mischung aus 2 ml NMP, 33 µlDiisopropylcarbodiimid (DIC), 62,0 mg Fmoc-β-Ala-OH, 27,0 mg HOAt eingeschweißt und 3 h bei 37°C inkubiert.
- Reaktionszyklus:: - Capping in 20 ml DMF + 600 µl Essigsäureanhydrid; 30"
- Capping in 20 ml DMF+600 µl Essigsäureanhydrid; 2'
- 2 x waschen in DMF, 2' und 5'
- 2 x waschen in Ethanol, 2' und 5'
- 2 x waschen in DMF, 2' und 5'
- entschützen in 20% Piperidin in DMF
- 2 x waschen in DMF, 2' und 5'
- 2 x waschen in Ethanol, 2' und 5'
- 2 x waschen in DMF, 2' und 5'
- Färben in 20 ml DMF + 600 µl BPB-Stammlösung, 10 mg/ml
- Entfärben in Ethanol
- trocknen

Aus allen zu spottenden Aminosäuren wurden 0,3 molare Stammlösungen in NMP hergestellt und über Molsieb bei 4°C aufbewahrt.

Vor dem Spotten wurde die jeweilige Stammlösung aufgewärmt und im Verhältnis 1:1:1 mit 0,3 molarer HOAt-Stammlösung und 0,4 molarer DIC-Stammlösung gemischt. Von dieser Mischung wurden 0,2 µl pro Spot auf die Membran aufgetragen. Dies wurde 3 x wiederholt, die Reaktionszeit nach jedem Auftragen betrug jeweils 20-40 min.
Gespottet wurde ein 8x12 - Raster im Mikrotiterplattenformat.

Nach jeden Spotzyklus wurde der folgende, an die Apparatur gemäß Fig. 1 angepaßte Reaktionszyklus gefahren:
- Capping in 20 ml DMF+600 µl Essigsäureanhydrid (+ 600 µl Pyridin); 200 µl pro Spot; 10' Reaktionszeit, absaugen
- 2 x waschen in DMF; 200 µl pro Spot; permanent absaugen
- 2 x waschen in Ethanol; permanent absaugen
- 2 x waschen in DMF; permanent absaugen
- entschützen in 20 % Piperidin in DMF; 10' Reaktionszeit, absaugen
- 2 x waschen in DMF; 200 µl pro Spot; permanent absaugen
- 2 x waschen in Ethanol; permanent absaugen
- mindestens 45' trockensaugen.

Folgende Linkermoleküle werden aufgetragen:
- Fmoc-Rink-Handle (in Array 1, Zeilen 1-4) bzw. Fmoc-β-Ala-OH (in Array 2 und 3, Zeilen 5-8 und 9-12)
- Fmoc-Lys(Dansyl)-OH.

Danach wurden 10 Zyklen mit Fmoc-A(aeg)-OH (Zeilen 1, 5, 9); Fmoc-C(aeg)-OH (Zeilen 2, 6, 10); Fmoc-G(aeg)-OH (Zeilen 3, 7, 11) bzw. Fmoc-T(aeg)-OH (Zeilen 4, 8 12) gespottet.

Die Membran wurde in die 3 Arrays aufgeteilt, Array 1 und 3 eingefroren.

### Endbehandlung für Array 2:

Array 2 wurde 10 min bei RT in 9,5 ml TFA, 500 µl Triisobutylsilan inkubiert (Abspaltung der Seitenschutzgruppen), 2 x in DMF und 1 x in Ethanol gewaschen und getrocknet.

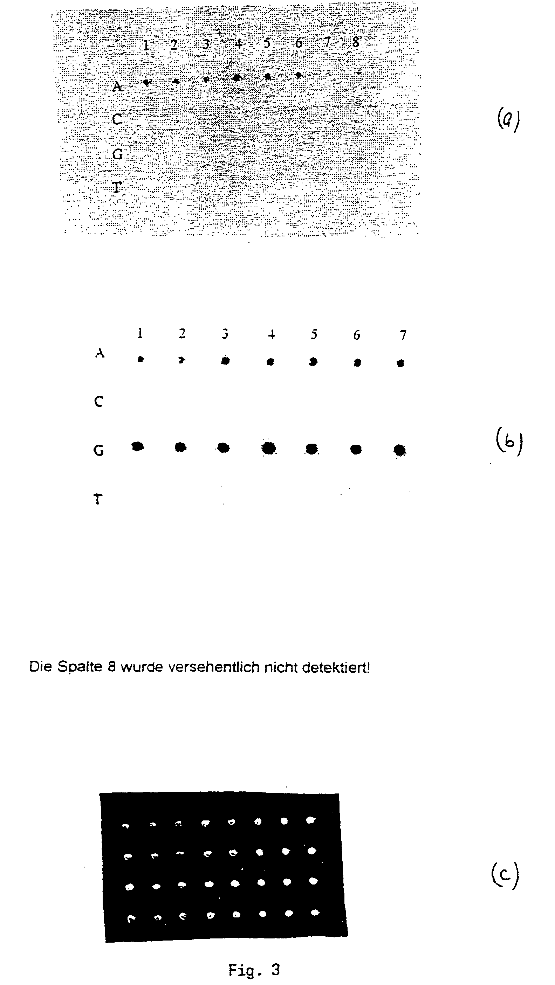

Danach wurde Array 2 in Hybridisierungslösung (d(T)₁₆ ³³PγATP) für 30' bei RT inkubiert und gewaschen. Die Membran wurde dann über Nacht auf einem Phos-phorimager-Screen exponiert. Es zeigte sich das in Fig. 3a gezeigte Bild.

Die Membran wurde anschließend gewaschen und zusätzlich in Hybridisierungslösung (d(C)₁₆ ³³PγATP) für 30' bei RT inkubiert, gewaschen und für 4 Tage auf einen Phosphorimager-Screen exponiert. Die Signale sind deutlich wie in Fig. 3b gezeigt.

Die Effizienz der ersten Kopplung kann anhand der Fluoreszenz-Intensität des ersten ankondensierten Bausteins ermittelt werden. In diesem Fall wurde Fmoc-Lys(Dansyl)-OH als erster Baustein eingesetzt. Wird dieser Fluoreszenz-Label während der Synthese an bestimmten Punkten nur eingesetzt, kann über die relative Intensität die Kondensationsausbeute bestimmt werden. Es ergibt sich bei 353 nm (normale UV-Lampe) das in Fig. 3c gezeigte Bild. Hieraus läßt sich erkennen, daß die Verteilung der Spots punktuell stattfand und keine Kreuzkontamination erfolgt.

## Patentansprüche

1. Durchflußeinrichtung mit mindestens dreiteiligem Aufbau und einer Abfolge der Teile I, II, III, wobei
- Teil I nebeneinander angeordnete Bohrlöcher enthält, die an ihrer Unterseite jeweils durch einen Dichtring abgedichtet sind,
- Teil II eine grobporige Membran; in einer 1. Vertiefung nebeneinander angeordnete Bohrlöcher, die jeweils durch einen Dichtring abgedichtet sind; und in einer 2. Vertiefung einen Saugkanal zum Anlegen eines Vakuums enthält,
- Teil III in einer 1. Vertiefung nebeneinander angeordnete Bohrlöcher, die jeweils durch einen Dichtring abgedichtet sind, und mindestens einen Anschluß zum Anlegen eines Vakuums enthält.

2. Durchflußeinrichtung nach Anspruch 1, wobei sich zwischen Teil I und Teil II eine Synthesemembran, die zur Bindung von Biopolymeren befähigt ist, befindet.

3. Durchflußeinrichtung nach Anspruch 2, wobei die Synthesemembran funktionalisiert ist.

4. Durchflußeinrichtung nach Anspruch 2 oder 3, wobei die Synthesemembran aus Nylon, Polyamid, Cellulose, Polypropylen, PFTE, Polyolefin, Polyethylen oder Polystyrol, Polyvinylidenfluorid, Glasfiber, PVC, Polymethylpenten oder Polynorbornen-Copolymere ist.

5. Durchflußeinrichtung nach Anspruch 3 oder 4, wobei die Synthesemembran Hydroxyl-, Amino-, Amid-, Phosphat-, Alkyl-, Halogen-, Carboxyl-, Carbonyl-, Thio-, Arylgruppen, Ethengruppen oder Ethingruppen aufweist.

6. Durchflußeinrichtung nach einem der vorhergehenden Ansprüche, wobei Teil III zwei Anschlüsse zum Anlegen von Vakuum aufweist.

7. Durchflußeinrichtung nach einem der vorhergehenden Ansprüche, wobei die grobporige Membran in Teil II aus Polyethylen, Polypropylen, PFTE oder Delrin besteht.

8. Durchflußeinrichtung nach einem der vorhergehenden Ansprüche, wobei die Durchflußeinrichtung aus einem inerten Material aufgebaut ist.

9. Durchflußeinrichtung nach Anspruch 8, wobei das inerte Material Delrin, PTFE oder keramisches Material ist.

10. Verwendung der Durchflußeinrichtung nach einem der vorhergehenden Ansprüche zum Binden von Biopolymeren an Membranoberflächen.

11. Verwendung nach Anspruch 10, wobei es sich um den Aufbau von membrangebundenen Molekülbibliotheken handelt.

12. Verwendung nach Anspruch 10 oder 11, wobei die Biopolymeren bzw. Molekülbibliotheken DNA, RNA, Aminosäuren, Peptide, Proteinen, Nukleinsäureanaloga umfassen.

## Claims

1. A flow-through device having at least a three-part design and a sequence of parts I, II, III, wherein
- part I contains drilled holes arranged side by side and sealed on their bottom side by a sealing ring each,
- part II contains a coarsely porous membrane; drilled holes arranged in a 1^{st} recess side by side and sealed by a sealing ring each; and a suction passage or channel in a 2^{nd} recess for applying a vacuum,
- part III includes drilled holes arranged in a 1^{st} recess side by side and sealed by a sealing ring each, and at least one connection for applying a vacuum.

2. The flow-through device according to claim 1, wherein a synthesis membrane capable of binding biopolymers is disposed between part I and part II.

3. The flow-through device according to claim 2, wherein the synthesis membrane is functionalized.

4. The flow-through device according to claim 2 or 3, wherein the synthesis membrane is made of nylon, polyamide, cellulose, polypropylene, PTFE, polyolefin, polyethylene or polystyrene, polyvinylidene fluoride, glass fiber, PVC, polymethylpentene, or polynorbornene copolymer.

5. The flow-through device according to claim 3 or 4, wherein the synthesis membrane comprises hydroxyl, amino, amide, phosphate, alkyl, halogen, carboxyl, carbonyl, thio, aryl groups, ethene groups or ethyne groups.

6. The flow-through device according to any one of the preceding claims, wherein part III includes two connections for applying a vacuum.

7. The flow-through device according to any one of the preceding claims, wherein the coarsely porous membrane in part II consists of polyethylene, polypropylene, PTFE or Delrin.

8. The flow-through device according to any one of the preceding claims, wherein the flow-through device is composed of an inert material.

9. The flow-through device according to claim 8, wherein the inert material is Delrin, PTFE or ceramic material.

10. Use of the flow-through device according to any one of the preceding claims for binding biopolymers to membrane surfaces.

11. Use according to claim 10, wherein the design of membrane-bound molecule libraries is concerned.

12. Use according to claim 10 or 11, wherein the biopolymers or molecule libraries comprise DNA, RNA, amino acids, peptides, proteins, nucleic acid analogs.

## Revendications

1. Dispositif à flux continu à structure au moins tripartite et à succession des parties I, II et III, dans lequel :
la partie I comprend des puits disposés côte à côte, dont chacun est rendu étanche à sa partie inférieure par un joint d'étanchéité ;
la partie II comprend une membrane à larges pores, des puits disposés côte à côte dans un premier renfoncement dont chacun est rendu étanche par un joint d'étanchéité, et un canal de succion dans un second renfoncement pour la création d'un vide ;
la partie III comprend des puits disposés côte à côte dans un premier renfoncement dont chacun est rendu étanche par un joint d'étanchéité, et au moins un raccord pour la création d'un vide.

2. Dispositif à flux continu suivant la revendication 1, dans lequel une membrane de synthèse rendue apte à la fixation de biopolymères se trouve entre la partie I et la partie II.

3. Dispositif à flux continu suivant la revendication 2, dans lequel la membrane de synthèse est fonctionnalisée.

4. Dispositif à flux continu suivant la revendication 2 ou 3, dans lequel la membrane de synthèse est en Nylon, polyamide, cellulose, polypropylène, PFTE, polyoléfine, polyéthylène ou polystyrène, polyfluorure de vinylidène, fibre de verre, PVC, polyméthylpentène ou copolymères de polynorbornène.

5. Dispositif à flux continu suivant la revendication 3 ou 4, dans lequel la membrane de synthèse porte des groupes hydroxyle, amino, amide, phosphate, alkyle, halogéno, carboxyle, carbonyle, thio, des groupes aryle, des groupes éthène ou des groupes éthyne.

6. Dispositif à flux continu suivant l'une des revendications précédentes, dans lequel la partie III présente deux raccords pour la création du vide.

7. Dispositif à flux continu suivant l'une des revendications précédentes, dans lequel la membrane à larges pores dans la partie II est en polyéthylène, polypropylène, PFTE ou Delrin.

8. Dispositif à flux continu suivant l'une des revendications précédentes, dispositif qui est réalisé en un matériau inerte.

9. Dispositif à flux continu suivant la revendication 8, dans lequel le matériau inerte consiste en Delrin, en PTFE ou en un matériau céramique.

10. Utilisation du dispositif à flux continu suivant l'une des revendications précédentes pour la fixation de biopolymères aux surfaces de membranes.

11. Utilisation suivant la revendication 10, dans laquelle il s'agit de la réalisation de bibliothèques moléculaires liées à des membranes.

12. Utilisation suivant la revendication 10 ou 11, dans laquelle les biopolymères ou les bibliothèques moléculaires comprennent de l'ADN, de l'ARN, des aminoacides, des peptides, des protéines, des analogues d'acides nucléiques.
